# EUROPÄISCHE PATENTSCHRIFT

(11) **EP 2 427 241 B1**
(45) Veröffentlichungstag und Bekanntmachung des Hinweises auf die Patenterteilung: **10.10.2012**
(21) Anmeldenummer: 10720766.4
(22) Anmeldetag: 17.05.2010
(51) Int. Cl.: A61M 16/04, A61F 2/20

(54) **FILTERVORRICHTUNG ZUR VERWENDUNG BEI EINEM TRACHEOSTOMA**
FILTER DEVICE FOR USE IN A TRACHEOSTOMA
DISPOSITIF FILTRANT POUR L'UTILISATION DANS UN TRACHÉOSTOME

(43) Veröffentlichungstag der Anmeldung: 14.03.2012
(73) Patentinhaber: Servona GmbH, 53842 Troisdorf (DE)
(72) Erfinder: WEINITSCHKE, Bodo, 53842 Troisdorf (DE)
(74) Vertreter: von Kreisler Selting Werner
(86) Internationale Anmeldenummer: PCT/EP2010/056745
(87) Internationale Veröffentlichungsnummer: WO 2011/144237

(56) Entgegenhaltungen:
- EP-B1- 1 077 658
- WO-A2-2008/132222
- US-A- 5 738 095

## Beschreibung

Die Erfindung betrifft eine Filtervorrichtung zur Verwendung bei einem Tracheostoma.

Halsatmer weisen ein Tracheostoma auf, durch das sie ein- und ausatmen. Das Tracheostoma kann mit einer Filtervorrichtung versehen sein, die neben der reinen Filterfunktion auch zu einer Luftbefeuchtung und Lufterwärmung führt. Derartige Filtervorrichtungen sind unter der Bezeichnung HME (Heat and Moisture Exchanger) bekannt und weisen ein Gehäuse auf, das in eine Fassung bzw. einen Kragen eingesetzt wird, welche/welcher das Tracheostoma umgibt und mit Hilfe eines Klebepflasters (auch Basisplatte genannt) auf der Haut um das Tracheostoma herum fixiert ist. Es ist auch möglich, die Filtervorrichtung in eine Trachealkanüle oder ein ähnliches an und/oder in einem Tracheostoma verwendbares medizinisches Hilfsmittel einzusetzen. Beispiele für Filtervorrichtungen der zuvor genannten Art finden sich in EP-B-0 735 844 (DE-T-694 05 587) und US-A-5 738 095, WO-A-2008/132222 sowie EP-B-1 077 658 (DE-T-699 20 440). Eine weitere bekannte Filtervorrichtung ist in EP-B-0 078 685 und US-B-7 025 784 beschrieben.

Um einem Halsatmer, dem der Kehlkopf entfernt worden ist, dennoch das Sprechen zu ermöglichen, kann zwischen Luft- und Speiseröhre ein sogenanntes Shunt-Ventil eingesetzt werden. Über dieses Ventil wird die von der Lunge kommende Luft zum Mund/-Rachenraum geführt, wenn das Tracheostoma blockiert wird. Filtervorrichtungen zur Verwendung bei einem Tracheostoma weisen daher mitunter auch die Möglichkeit des Verschlusses auf.

Die bekannten Filtervorrichtungen haben sich in der Praxis grundsätzlich bewährt. Im Hinblick auf eine zuverlässige Filterfunktion sowie ausreichende Luftbefeuchtung sowie -erwärmung ist es zweckmäßig, wenn das Filtervolumen in Relation zur Gesamtbaugröße der Filtervorrichtung so groß wie möglich ist.

Aufgabe der Erfindung ist es, eine Filtervorrichtung zur Verwendung bei einem Tracheostoma zu schaffen, die über ein möglichst großes Filterkörpervolumen verfügt.

Zur Lösung dieser Aufgabe wird mit der Erfindung eine Filtervorrichtung zur Verwendung bei einem Tracheostoma vorgeschlagen, die versehen ist mit den Merkmalen des Anspruchs 1. Die Unteransprüche betreffen einzelne Ausgestaltungen der Erfindung.

Die erfindungsgemäße vorzugsweise aus Kunststoff und insbesondere aus thermoplastischen Kunststoff bestehende Filtervorrichtung weist ein Gehäuse auf, das mit einer ersten und einer zweiten Öffnung für jeweils einzuatmende und auszuatmende Luft versehen ist. Dabei ist die erste Öffnung an dem im benutzten Zustand der Filtervorrichtung dem Tracheostoma zugewandten Ende des Gehäuses angeordnet. Dieses Ende des Gehäuses befindet sich beispielsweise in einer Fassung bzw. einem Kragen, der von einer Klebefolie absteht, welche auf die Haut um das Tracheostoma herum aufgeklebt ist, oder z. B. in einer Aufnahme einer Trachealkanüle. Innerhalb des Gehäuses befindet sich ein bewegbares Verschlusselement, das die erste Öffnung wahlweise verschließt. Die Bewegung des Verschlusselements aus der Öffnungsposition in die Verschlussposition erfolgt mit Hilfe eines Betätigungselements, das vorzugsweise als Drucktaste oder anderweitiges platten- bzw. scheibenförmiges Element ausgebildet ist. Das Betätigungselement ist mit dem Verschlusselement starr verbunden. Auf das Betätigungselement wirkt direkt oder indirekt ein Federelement ein, das sich am Gehäuse abstützt bzw. mit diesem verbunden oder auf andere Art und Weise an diesem befestigt ist und das Betätigungselement und damit das Verschlusselement in die Öffnungsposition des Verschlusselements vorspannt. Zwischen dem Verschlusselement und dem Betätigungselement befindet sich ein Filterkörper, der vorzugsweise aus Schaumstoff besteht und den gesamten Raum oder einen Teil des Raums zwischen dem Verschlusselement und dem Betätigungselement ausfüllt.

Erfindungsgemäß ist nun der Filterkörper mit einer Aufnahmeaussparung versehen, durch die sich hindurch das Federelement erstreckt.

Das Federelement befindet sich also innerhalb des Filterkörpers, womit der gesamte Raum zwischen dem Betätigungselement und dem Verschlusselement vom Filterkörper ausgefüllt sein kann. Damit ist das zur Verfügung stehende Volumen des Filterkörpers maximal groß, was für eine gute Filter-, Luftbefeuchtungs- und Lufterwärmungsfunktion von Vorteil ist.

In vorteilhafter Weiterbildung der Erfindung ist das Federelement als verformbarer, im Wesentlichen in Richtung auf das Betätigungselement vorgewölbter Federsteg ausgebildet. Beispielsweise erstreckt sich der Federsteg quer durch das Gehäuse hindurch und ist an seinen beiden gegenüberliegenden Enden an im Wesentlichen gegenüberliegenden Bereichen der Gehäusewand befestigt bzw. mit dem Gehäuse einteilig verbunden. Der Federbügelsteg steht in Richtung auf das Betätigungselement vor, wobei der Scheitelpunkt des Federbügelstegs mit dem Betätigungselement in Wirkverbindung steht. Beim Niederdrücken des Betätigungselements (und damit beim Bewegen des Verschlusselements aus dessen Öffnungsposition in dessen Schließposition) verformt sich der Federsteg, der anschließend beim Aufheben der auf das Betätigungselement wirkenden Kraft das Betätigungselement und damit das Verschlusselement wieder in ihre Ausgangs- bzw. Öffnungspositionen zurückbewegt.

Zweckmäßigerweise ist die Aufnahmeaussparung des Filterkörpers als mindestens zu einer Seite offene Nut ausgebildet. Der Filterkörper ragt zweckmäßigerweise bis in die zweite Gehäuseöffnung hinein.

In weiterer vorteilhafter Ausgestaltung der Erfindung ist vorgesehen, dass das Gehäuse im Wesentlichen zylinderförmig ist und an einem ersten axialen Ende einen die erste Öffnung definierenden Innenflansch aufweist, dass das Betätigungselement an dem der ersten Öffnung gegenüberliegenden zweiten axialen Ende des Gehäuses angeordnet ist und dass die zylindrische Wand des Gehäuses an dessen zweiten axialen Ende mehrere die zweite Öffnung bildende Aussparungen aufweist. Während die erste Öffnung des Gehäuses an der ersten axialen (Stirn-)Seite des zylindrischen Gehäuses angeordnet ist, befindet sich die zweite Öffnung an dem zweiten axialen Ende der zylindrischen Wand des Gehäuses und ist aus mehreren dort angeordneten Aussparungen gebildet. Die (Stirn-)Seite des Gehäuses an dessen zweiten axialen Ende ist mit dem Betätigungselement versehen, das bei Betätigung in das Gehäuse hinein bewegt wird. Der Filterkörper erstreckt sich vorzugsweise bis zum Betätigungselement, liegt also den längs des Umfangs am zweiten axialen Ende des Gehäuses ausgebildeten, die zweite Öffnung bildenden Aussparungen gegenüber. Beim Niederdrücken des Betätigungselements wird dieses an den Aussparungen der zweiten Öffnung vorbeibewegt. Weder bei dieser Ausgestaltung des Gehäuses noch bei der erfindungsgemäßen Filtervorrichtung im Allgemeinen wird der Filterkörper durch die Betätigung des Betätigungselements komprimiert sondern bewegt sich mit dem Betätigungselement und dem Verschlusselement in Richtung auf die erste Öffnung; denn die Kraft, die bei Betätigung des Betätigungselements auf dieses einwirkt, wird über die starre Verbindung des Betätigungselements mit dem Verschlusselement auf dieses übertragen, womit das Verschlusselement aus der Öffnungsposition in die Schließposition überführt wird.

In weiterer vorteilhafter Ausgestaltung der Erfindung ist vorgesehen, dass das Gehäuse zweiteilig ausgeführt ist und mit einem ersten Gehäuseteil, in dem die erste Öffnung ausgebildet ist, und einem zweiten Gehäuseteil versehen ist, in dem die zweite Öffnung ausgebildet ist und das das Federelement aufweist, welches an dem zweiten Gehäuseteil gehalten ist und das Betätigungselement trägt.

Die Erfindung wird nachfolgend anhand eines Ausführungsbeispiels und unter Bezugnahme auf die Zeichnung näher erläutert. Im Einzelnen zeigen dabei:
- Fig. 1: eine Darstellung des Tragezustands einer Filtervorrichtung bei einem Halsatmer,
- Fig. 2: in Seitenansicht die Filtervorrichtung mit Klebeplatte und die Filtervorrichtung aufnehmendem Kragen bzw. aufnehmender Fassung,
- Fig. 3: einen Längsschnitt durch die Filtervorrichtung bei geöffneter erster Öffnung zum Ein- und Ausatmen durch den Halsatmer,
- Fig. 4: eine Explosionsdarstellung der Einzelteile der Vorrichtung gemäß Fig. 3 und
- Fig. 5: einen Längsschnitt durch die Filtervorrichtung bei verschlossener erster Öffnung.

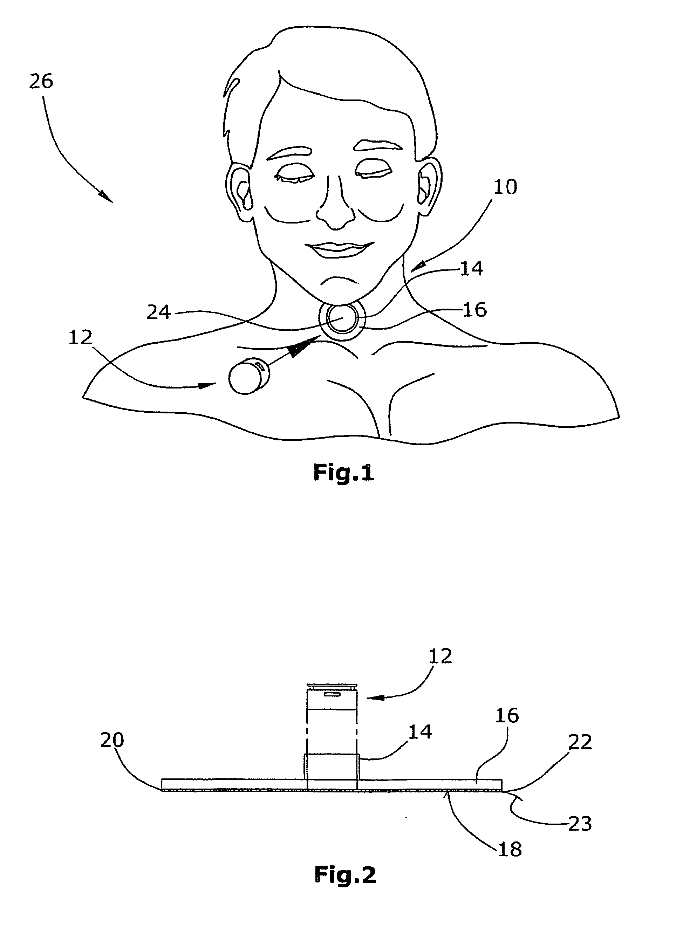

In den Fign. 1 und 2 ist die Verwendung eines Ausführungsbeispiels der erfindungsgemäßen Filtervorrichtung 10 gezeigt. Die Filtervorrichtung 10 weist ein im Wesentlichen zylinderförmiges Gehäuse 12 auf, das in einen Aufnahmekragen 14 eingesetzt wird, welcher von einer Folie bzw. flexiblen Platte 16 absteht, die an ihrer Unterseite 18 mit einer Klebeschicht 20 versehen ist, die wiederum vor dem Gebrauch durch eine Schutzfolie 22 mit Anfassende 23 abgedeckt ist.

Die Folie bzw. Platte 16 wird auf der Haut um das Tracheostoma 24 eines Halsatmers 26 herum aufgeklebt, wobei der Kragen 14 mit dem Tracheostoma 24 fluchtet. Das Gehäuse 12 der Filtervorrichtung 10 kann nun in den Kragen 14 eingesetzt werden, wobei die Filtervorrichtung 10 nach Gebrauch bequem gegen eine neue Filtervorrichtung 10 ausgetauscht werden kann.

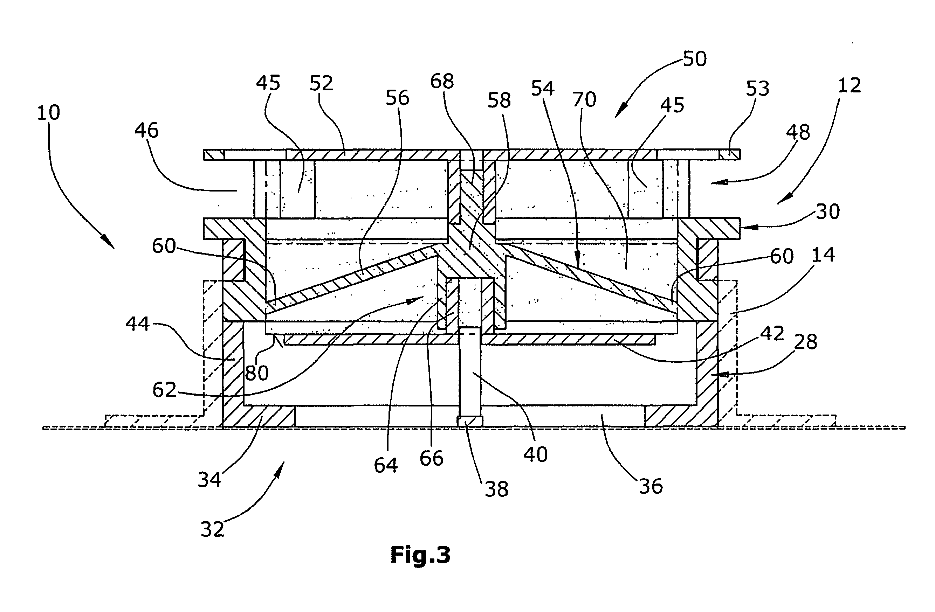

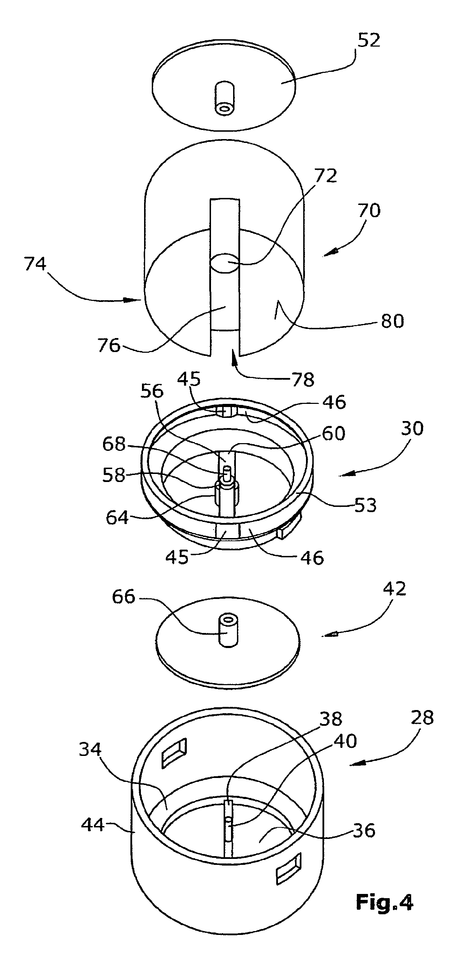

Der Aufbau und die Konstruktion der Filtervorrichtung 10 sind in den Fign. 3 bis 5 näher gezeigt. Das im Wesentlichen zylindrische Gehäuse 12 weist ein erstes Gehäuseteil 28 und ein zweites Gehäuseteil 30 auf, die z.B. ineinandergesteckt und miteinander verrastet sind. Das erste Gehäuseteil 28 weist an dem ersten axialen Ende 32 des Gehäuses 12 einen Innenflansch 34 auf, der eine erste Öffnung 36 zum Ein- und Ausatmen definiert. Diese erste Öffnung 36 ist von einem Steg 38 durchzogen, von dem zum Innern des Gehäuses 12 ein Führungsstift 40 absteht, auf welchem ein platten- bzw. scheibenförmiges Verschlusselement 42 geführt ist.

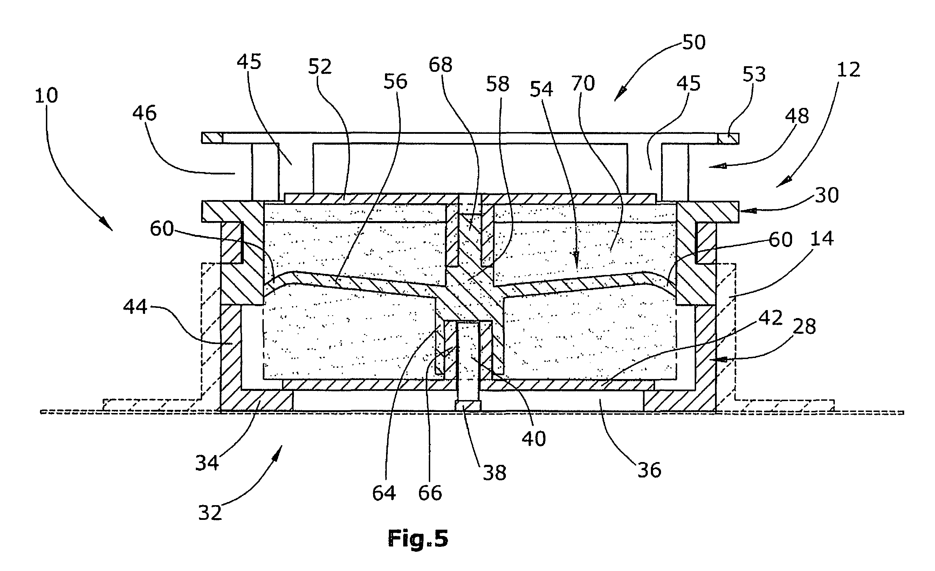

Das zweite Gehäuseteil 30 weist in Verlängerung der zylindrischen Gehäusewand 44 des ersten Gehäuseteils 28 mehrere durch Stege 45 getrennte Aussparungen 46 auf, die eine zweite Öffnung 48 zum Ein- und Ausatmen bilden. Diese Aussparungen 46 sind in der zylindrischen Wand des Gehäuses 12 im Bereich von dessen zweiten axialen Ende 50 angeordnet. Am zweiten axialen Ende 50 des Gehäuses 12 ist ein platten- bzw. scheibenförmiges Betätigungselement 52 zur manuellen Betätigung des Verschlusselements 42 angeordnet. Das Betätigungselement 52, um das sich ein Ring 53 des zweiten Gehäuseteils 30 erstreckt, ist starr mit dem Verschlusselement 42 gekoppelt, so dass das Verschlusselement 42 beim Niederdrücken des Betätigungselements 52 aus der Öffnungsposition gemäß Fig. 3, in der es die erste Öffnung 36 freigibt, in die Verschließposition gemäß Fig. 5 überführt wird, in der es die erste Öffnung 36 verschließt. Die Bewegung aus der Verschließposition zurück in die Öffnungsposition erfolgt automatisch mit Hilfe eines beim Niederdrücken des Betätigungselements 52 sich spannenden Federelements 54, das als zum Betätigungselement 52 hin vorstehender Federsteg 56 ausgebildet ist. Der Federsteg 56 ist im Bereich seines Scheitelpunktes 58 mit dem Betätigungselement 52 verbunden, während seine beiden Enden 60 mit dem zweiten Gehäuseteil 30 verbunden sind. Die starre Kopplung des Betätigungselements 52 mit dem Verschlusselement 42 erfolgt durch einen Stößel 62, der einen ersten sich vom Federsteg 56 im Bereich von dessen Scheitelpunkt 58 aus erstreckenden ersten Stößelteil 64 und einen mit diesem in Eingriff befindlichen zweiten Stößelteil 66 aufweist, der von dem Verschlusselement 42 aufragt. Vom Scheitelpunkt 58 des Federsteges 56 aus erstreckt sich in Richtung auf das Betätigungselement 52 ein Stift 68, der das Betätigungselement 52 trägt.

Der Bereich (das Volumen) innerhalb des Gehäuses 12 zwischen dem Verschlusselement 42 und dem Betätigungselement 52 bzw. zwischen dem Verschlusselement 42 und den die zweite Öffnung 48 bildenden Aussparungen 46 ist von einem zylindrischen Filterkörper 70 ausgefüllt, der beispielsweise aus Schaumstoffmaterial besteht. Der Filterkörper 70 bewegt sich zusammen mit dem Betätigungselement 52 und dem Verschlusselement 42 in dem Gehäuse 12, wenn das Betätigungselement 52 niedergedrückt wird bzw. wenn das Federelement 54 das Verschlusselement 42 wieder zurückbewegt.

Wie anhand der Figuren zu erkennen ist, erstrecken sich das Federelement 54 und der Stößel 62 durch den Filterkörper 70 hindurch. Zu diesem Zweck weist der Filterkörper 70 eine axiale Bohrung 72 zur Aufnahme des Stößels 62 und in seinem dem Verschlusselement 42 zugewandten unteren Bereich 74 eine Aufnahmenut 76 als Aufnahmeaussparung 78 für den Federsteg 56 auf. Die Aufnahmeaussparung 78 ist zur Unterseite 80 des Filterkörpers 70 offen. Durch die Integration des Federmechanismus in den Filterkörper 70 ist es möglich, das Filtermaterialvolumen der Filtervorrichtung 10 trotz geringer Bauhöhe zu maximieren. Dies ist im Hinblick auf eine zuverlässige und ausreichende Filter-, Luftbefeuchtungs- und Lufterwärmungsfunktion von Vorteil.

## Patentansprüche

1. Filtervorrichtung zur Verwendung bei einem Tracheostoma, mit
- einem Gehäuse (12), das eine in Fluidverbindung mit dem Tracheostoma bringbare erste Öffnung (36) und eine zweite Öffnung (48) aufweist,
- einem Verschlusselement (42), das innerhalb des Gehäuses (12) zwischen einer Verschließposition, in der das Verschlusselement (42) die erste Öffnung (36) verschließt, und einer Öffnungsposition, in der das Verschlusselement (42) die erste Öffnung (36) freigibt, bewegbar ist,
- einem mit dem Verschlusselement (42) verbundenen Betätigungselement (52) zur Betätigung des Verschlusselements (42),
- einem in dem Gehäuse (12) angeordneten Filterkörper (70) und
- einem einerseits mit dem Betätigungselement (52) und andererseits mit dem Gehäuse (12) im Bereich zwischen dem Verschlusselement (42) und der zweiten Öffnung (48) in Wirkverbindung stehendes Federelement (54) zum automatischen Bewegen des Verschlusselements (42) in dessen Öffnungsposition,
**dadurch gekennzeichnet,**
- **dass** der Filterkörper (70) wenigstens eine Aufnahmeaussparung (78) aufweist, durch die sich das Federelement (54) erstreckt.

2. Filtervorrichtung nach Anspruch 1, **dadurch gekennzeichnet, dass** der Filterkörper (70) zwischen dem Verschlusselement (42) und dem Betätigungselement (52) angeordnet ist.

3. Filtervorrichtung nach Anspruch 1 oder 2, **dadurch gekennzeichnet, dass** das Federelement (54) einen verformbaren, im Wesentlichen in Richtung auf das Betätigungselement (52) vorgewölbten Federsteg (56) aufweist, dessen Scheitelpunkt (58) in Wirkverbindung mit dem Betätigungselement (52) steht und dessen beide Enden innen an im Wesentlichen gegenüberliegenden Bereichen einer Wand des Gehäuses (12) befestigt sind.

4. Filtervorrichtung nach Anspruch 3, **dadurch gekennzeichnet, dass** die Aufnahmeaussparung (78) des Filterkörpers (70) als zumindest zu einer Seite offene Nut (76) ausgebildet ist.

5. Filtervorrichtung nach einem der Ansprüche 1 bis 4, **dadurch gekennzeichnet, dass** das Gehäuse (12) im Wesentlichen zylinderförmig ist und an einem ersten axialen Ende (32) einen die erste Öffnung (36) definierenden Innenflansch (34) aufweist, dass das Betätigungselement (52) an dem der ersten Öffnung (36) gegenüberliegenden zweiten axialen Ende (50) des Gehäuses (12) angeordnet ist und dass die zylindrische Wand des Gehäuses (12) an dessen zweiten axialen Ende mehrere die zweite Öffnung (48) bildende Aussparungen (46) aufweist.

6. Filtervorrichtung nach einem der Ansprüche 1 bis 5, **dadurch gekennzeichnet, dass** das Betätigungselement (52) und das Verschlusselement (42) jeweils plattenförmig und insbesondere jeweils scheibenförmig ausgebildet sind.

7. Filtervorrichtung nach Anspruch 6, **dadurch gekennzeichnet, dass** das Betätigungselement (52), das Federelement (54), das Verschlusselement (42) und das Gehäuse (12) insbesondere thermoplastischen Kunststoff aufweisen und/oder dass der Filterkörper (70) Schaumstoff aufweist.

8. Filtervorrichtung nach einem der Ansprüche 1 bis 7, **dadurch gekennzeichnet, dass** das Gehäuse (12) zweiteilig ausgeführt ist und mit einem ersten Gehäuseteil (28), in dem die erste Öffnung (36) ausgebildet ist, und einem zweiten Gehäuseteil (30) versehen ist, in dem die zweite Öffnung (48) ausgebildet ist und das das Federelement (54) aufweist, welches an dem zweiten Gehäuseteil(30) gehalten ist und das Betätigungselement (52) trägt.

## Claims

1. A filter device for use in a tracheostoma, comprising
- a housing (12) having a first opening (36) adapted to be brought into fluid connection with the tracheostoma, and a second opening (48),
- a closure element (42) which is moveable within the housing (12) between a closing position in which the closure element (42) closes the first opening (36), and an opening position in which the closure element (42) clears the first opening (36),
- an actuating element (52) connected to the closure element (42) for operating the closure element (42),
- a filter body (70) arranged in the housing (12), and
- a spring element (54) being operatively connected, on the one hand, to the actuating element (52) and, on the other hand, to the region of the housing (12) between the closure element (42) and the second opening (48), said spring element being provided for automatically moving the closure element (42) into its opening position,
**characterized in**
- **that** the filter body (70) comprises at least one receiving recess (78) having the spring element (54) extending through it.

2. The filter device according to claim 1, **characterized in that** the filter body (70) is arranged between the closure element (42) and the actuating element (52).

3. The filter device according to claim 1 or 2, **characterized in that** the spring element (54) comprises a deformable spring bar (56) bulging substantially toward the actuating element (52), said spring bar being by its apex (58) operatively connected to the actuating element (52) and having its two ends fastened inside to substantially opposite regions of the wall of the housing (12).

4. The filter device according to claim 3, **characterized in that** the receiving recess (78) of the filter body (70) is formed as a groove (76) being open at least to one side.

5. The filter device according to any one of claims 1 to 4, **characterized in that** the housing (12) is substantially cylindrical and at a first axial end (32) comprises an inner flange (34) defining the first opening (36), that the actuating element (52) is arranged at the second axial end (50) of the housing (12) opposite to the first opening (36) and that the cylindrical wall of the housing (12) at the second axial end thereof comprises a plurality of recesses (46) forming the second opening (48).

6. The filter device according to any one of claims 1 to 5, **characterized in that** the actuating element (52) and the closure element (42) are respectively plate-shaped and particularly respectively disk-shaped.

7. The filter device according to claim 6, **characterized in that** the actuating element (52), the spring element (54), the closure element (42) and the housing (12) comprise particularly thermoplastic synthetic material and/or that the filter body (70) comprises foamed material.

8. The filter device according to any one of claims 1 to 7, **characterized in that** the housing (12) is of a two-part configuration and comprises a first housing portion (28) having the first opening (36) formed therein, and a second housing portion (30) having the second opening (48) formed therein and comprising the spring element (54), the spring element being held on the second housing portion (30) and carrying the actuating element (52).

## Revendications

1. Dispositif filtrant pour l'utilisation dans un trachéostome, avec
- un boîtier (12) qui présente une première ouverture (36), qui peut être amenée en liaison fluide avec le trachéostome, et une deuxième ouverture (48),
- un élément de fermeture (42) qui, à l'intérieur du boîtier (12), peut être déplacé entre une position de fermeture dans laquelle l'élément de fermeture (42) ferme la première ouverture (36), et une position d'ouverture dans laquelle l'élément de fermeture (42) libère la première ouverture (36),
- un élément d'actionnement (52) raccordé à l'élément de fermeture (42) pour l'actionnement de l'élément de fermeture (42),
- un corps filtrant (70) disposé dans le boîtier (12) et
- un élément à ressort (54) qui est en liaison opératoire d'un côté avec l'élément d'actionnement (52) et d'un autre côté avec le boîtier (12) dans la zone entre l'élément de fermeture (42) et la deuxième ouverture (48), et qui est destiné au déplacement automatique de l'élément de fermeture (42) dans sa position d'ouverture,
**caractérisé en ce que**
- le corps filtrant (70) présente au moins un évidement de réception (78) à travers lequel s'étend l'élément à ressort (54).

2. Dispositif filtrant selon la revendication 1, **caractérisé en ce que** le corps filtrant (70) est disposé entre l'élément de fermeture (42) et l'élément d'actionnement (52).

3. Dispositif filtrant selon la revendication 1 ou 2, **caractérisé en ce que** l'élément à ressort (54) présente une barrette à ressort (56) déformable pré-cintrée essentiellement en direction de l'élément d'actionnement (52), dont le point de sommet (58) est en liaison opératoire avec l'élément d'actionnement (52) et dont les deux extrémités sont fixées intérieurement au niveau de zones, essentiellement opposées, d'une paroi du boîtier (12).

4. Dispositif filtrant selon la revendication 3, **caractérisé en ce que** l'évidement de réception (78) du corps filtrant (70) est constitué en tant que rainure (76) ouverte au moins d'un côté.

5. Dispositif filtrant selon l'une des revendications 1 à 4, **caractérisé en ce que** le boîtier (12) est essentiellement de forme cylindrique et présente, au niveau d'une première extrémité axiale (32), une bride intérieure (34) définissant la première ouverture (36), **en ce que** l'élément d'actionnement (52) est disposé au niveau de la deuxième extrémité axiale (50) du boîtier (12) opposée à la première ouverture (36), et **en ce que** la paroi cylindrique du boîtier (12) présente, au niveau de sa deuxième extrémité axiale, plusieurs évidements (46) formant la deuxième ouverture (48).

6. Dispositif filtrant selon l'une des revendications 1 à 5, **caractérisé en ce que** l'élément d'actionnement (52) et l'élément de fermeture (42) sont constitués respectivement en forme de plaque et en particulier respectivement en forme de rondelle plate.

7. Dispositif filtrant selon la revendication 6, **caractérisé en ce que** l'élément d'actionnement (52), l'élément à ressort (54), l'élément de fermeture (42) et le boîtier (12) présentent en particulier une matière thermoplastique et/ou **en ce que** le corps filtrant (70) présente de la mousse synthétique.

8. Dispositif filtrant selon l'une des revendications 1 à 7, **caractérisé en ce que** le boîtier (12) est réalisé en deux parties et est muni d'une première partie de boîtier (28) dans laquelle est constituée la première ouverture (36) et d'une deuxième partie de boîtier (30) dans laquelle est constituée la deuxième ouverture (48) et qui présente l'élément à ressort (54) qui est retenu au niveau de la deuxième partie de boîtier (30) et qui porte l'élément d'actionnement (52).
